(19) Europäisches Patentamt
European Patent Office
Office européen des brevets

(11) Veröffentlichungsnummer: **0 093 329**
**B1**

(12) # EUROPÄISCHE PATENTSCHRIFT

(45) Veröffentlichungstag der Patentschrift: **29.01.86**

(51) Int. Cl.[4]: **G 03 G 5/06,** C 07 D 249/06

(21) Anmeldenummer: **83103860.9**

(22) Anmeldetag: **20.04.83**

(54) **Elektrographische Aufzeichnungsmaterialien mit speziellen Ladungsträger transportierenden Verbindungen.**

(30) Priorität: **29.04.82 DE 3215967**

(43) Veröffentlichungstag der Anmeldung: **09.11.83 Patentblatt 83/45**

(45) Bekanntmachung des Hinweises auf die Patenterteilung: **29.01.86 Patentblatt 86/5**

(84) Benannte Vertragsstaaten: **BE CH DE FR GB IT LI NL**

(56) Entgegenhaltungen:
**DE - A - 2 712 496**
**DE - B - 1 060 260**
**DE - B - 1 116 057**

(73) Patentinhaber: **BASF Aktiengesellschaft, Carl-Bosch-Strasse 38, D-6700 Ludwigshafen (DE)**

(72) Erfinder: **Eilingsfeld, Heinz, Dr., Pierstrasse 9 A, D-6710 Frankenthal (DE)**
Erfinder: **Etzbach, Karl-Heinz, Dr., Bensheimer Ring 9A, D-6710 Frankenthal (DE)**
Erfinder: **Hoffmann, Gerhard, Dr., Pappelstrasse 22, D-6701 Otterstadt (DE)**
Erfinder: **Neumann, Peter, Dr., Franz-Schubert-Strasse 1, D-6908 Wiesloch (DE)**



Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

**Beschreibung**

Die Erfindung betrifft elektrophotographische Aufzeichnungsmaterialien mit elektrisch leitenden Trägern, Ladungsträger erzeugenden Verbindungen bzw. Sensibilisatoren und speziellen Ladungsträger transportierenden Verbindungen.

Elektrophotographische Verfahren, dafür benötigte Materialien und verschiedene Varianten für den Aufbau von Aufzeichnungsmaterialien sind bekannt. Vorteilhaft für den Einsatz im Reproduktionssektor sind Materialien aus polymeren Bindemitteln, die an spezielle Anforderungen des jeweiligen Einsatzgebietes angepasst werden können, niedermolekulare organische Verbindungen, die in den Bindemitteln auch in höheren Konzentrationen löslich und zu einem Transport von Ladungsträgern des elektrischen Stromes befähigt sind, sowie Verbindungen, insbesondere Farbstoffe oder Pigmente, die durch Absorption des bildmässig eingestrahlten, aktinischen Lichts Ladungsträger des elektrischen Stromes erzeugen und diese unter Mithilfe des von aussen durch die elektrostatische Oberflächenladung aufgeprägten elektrischen Feldes auf die Ladung transportierenden Verbindungen übertragen können. Diese Ladungsträger erzeugenden Verbindungen können je nach Einsatzgebiet des Aufzeichnungsmaterials als eigene Schicht innerhalb einer Kompositstruktur eingebracht werden (vgl. DE-A-22 20 408) oder in Form monodispers gelöster Farbstoffmoleküle in der Mischung aus Bindemittel und Ladungsträger transportierenden Verbindungen vorhanden sein (vgl. DE-B-1 058 836). Das in der DE-A-22 20 408 beschriebene mehrlagige elektrophotographische Aufzeichnungsmaterial besteht aus einem elektrisch leitfähigen Trägermaterial, einer ersten, Farbstoff enthaltenden, etwa 0,005 bis 2 µm dicken, durch Belichtung mit aktinischem Licht Ladungsträger des elektrischen Stromes erzeugenden Schicht aus im Dunkeln isolierenden, organischen Materialien mit mindestens einer Ladungen transportierenden Verbindung.

Es ist auch bekannt, photohalbleitende organische Verbindungen zur Herstellung von elektrophotographischen Druckformen und insbesondere elektrophotographischen Offsetdruckformen zu verwenden (vgl. DE-B-1 117 391 und 1 120 875, DE-B-15 22 497 und 27 26 116).

Die gestiegenen Anforderungen an Reproduktionssysteme verlangen eine Vielfalt von Aufzeichnungsmaterialien und -systemen, um für spezielle Probleme optimale Lösungen aussuchen zu können. Gewünscht ist eine hohe Lichtempfindlichkeit, eine gute Auflösung und gute Betonerung. Die oft beanstandete ungenügende Betonerung, die auf eine ungünstige Feldstärkedifferenzierung zwischen belichteten und unbelichteten Flächen hinweist, ist hierbei oft auf eine zu hohe Dunkelleitfähigkeit des Aufzeichnungsmaterials im beladenen Zustand zurückzuführen, so dass eine ungenügende Oberflächenladungsdichte vor der aktinischen bildmässigen Belichtung vorliegt.

Der vorliegenden Erfindung lag die Aufgabe zugrunde, weitere elektrophotographische Aufzeichnungsmaterialien insbesondere für die Herstellung von elektrophotographischen Druckformen wie Offsetdruckformen zu entwickeln, die bei hoher Lichtempfindlichkeit, guter Auflösung und Verarbeitung gleichzeitig ein geringes Dunkelleitvermögen aufweisen.

Es wurde nun gefunden, dass man so verbesserte elektrophotographische Aufzeichnungsmaterialien mit elektrisch leitenden Trägern, Ladungsträger erzeugenden Verbindungen und Ladungsträger transportierenden Verbindungen erhält, wenn sie als Ladungsträger transportierende Verbindungen solche der Formel (I) enthalten

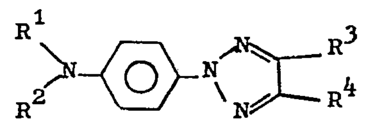 (I)

worin bedeuten:
$R^1$ und $R^2$ = Wasserstoff, Alkyl, Allyl, Benzyl oder ggf. subst. Phenyl, gleich oder verschieden, oder $R^1$ und $R^2$ gemeinsam

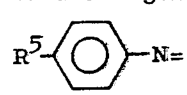 oder 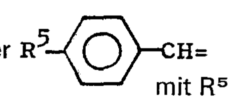
mit $R^5$ = $NR^1R^2$

$R^3$ = Alkyl oder Phenyl,
$R^4$ = Alkyl, Vinyl, Allyl, Carbalkoxy oder (Meth)-Acryloyl.

Bevorzugt sind hierbei die Verbindungen, bei denen in Formel (I)
$R^1$ = Alkyl, Benzyl oder Phenyl
$R^2$ = Alkyl, Benzyl oder Phenyl
$R^3$ = Alkyl oder Phenyl
$R^4$ = Alkyl oder Carbalkoxy bedeuten.

Bevorzugte Alkylreste enthalten 1 bis 4 C-Atome, bevorzugte Carbalkoxyreste sind der Carbmethoxy- und der Carbethoxyrest.

Beispiele sehr geeigneter Verbindungen sind:

(1) 2-(4'-Diethylaminophenyl)-4,5-dimethyltriazol-1,2,3,
(2) 2-(4'-Diethylaminophenyl)-4-phenyl-5-carbmethoxytriazol-1,2,3,
(3) 2-(4'-Diphenylaminophenyl)-4,5-dimethyltriazol-1,2,3,
(4) 2-(4'-Dibenzylaminophenyl)-4,5-dimethyltriazol-1,2,3,
(5) 2-(4'-Dibenzylaminophenyl)-4-methyl-5-carbmethoxytriazol-1,2,3,
(6) 2-(4'-Dimethylaminophenyl)-4,5-dimethyltriazol-1,2,3,
(7) 4-Diethylamino-4'-(4-methyl-5-carbmethoxytriazol-1,2,3-2-yl)-benzalanilin,
(8) 2-(4'-Diethylaminoazobenzol-4''-yl)-4,5-dimethyltriazol-1,2,3.

Besonders bewährt haben sich hiervon die Verbindungen (1)-(5).

Die erfindungsgemäss verwendeten substituierten Triazole sind nach bekannten Methoden der organischen Synthese herstellbar.

Die erfindungsgemäss verwendeten Ladungsträger transportierenden Verbindungen können mit Vorteil sowohl in einschichtig als auch in mehrschichtig auf elektroleitfähige Träger aufgebrachten Aufzeichnungssystemen verwendet werden.

Geeignete einschichtige Systeme nach Anspruch 4 weisen bevorzugt auf einem leitfähigen Trägermaterial eine Schicht aus (a) 45 bis 75 Gewichtsteilen eines Bindemittels, (b) 30 bis 60, insbesondere 35 bis 50 Gewichtsteilen einer der erfindungsgemäss verwendeten, Ladungsträger transportierenden Verbindungen, (c) ggf. 5 bis 25 Gewichtsteilen eines weiteren, im wesentlichen inaktiven Bindemittels und (d) 0,05 bis 0,8 Gewichtsteilen einer bei aktinischer Belichtung Ladungsträger erzeugenden Verbindung, insbesondere eines geeigneten Farbstoffs auf. Die Schichten werden mit Vorteil aus einer ca. 5 gew.%igen Lösung in einem geeigneten organischen Lösungsmittel auf das gereinigte leitfähige Trägermaterial so aufgebracht, dass nach dem Ablüften des Lösungsmittels eine Trockenschichtdicke von ca. 0,8 bis 40 µm (je nach Verwendungszweck, bei elektrophotographischen Druckformen insbesondere 0,8 bis 6 µm) resultiert.

Geeignete Mehrschichtsysteme nach Anspruch 3 haben bevorzugt auf einem elektroleitfähigen Trägermaterial z.B. (a) eine Ladungsträger erzeugende Schicht und (b) eine Ladungstransportschicht aus (b1) 30 bis 60 Gewichtsteilen mindestens einer Ladungsträger transportierenden Verbindung der Formel (I), (b2) 45 bis 75 Gewichtsteilen eines organischen Bindemittels und (b3) ggf. 5 bis 25 Gewichsteilen weiterer, die mechanischen Eigenschaften der Schicht verbessernden Zusätze. Die erste Schicht wird vorteilhaft in einer Dicke von 0,005 bis 5, insbesondere 0,1 bis 0,9 µm aus Lösung in einem geeigneten Lösungsmittel auf das Trägermaterial aufgetragen. Nach dem Auftrag erfolgt der Auftrag der zweiten Schicht in einer Dicke, dass nach dem Trocknen der Kompositstruktur eine Schichtdicke von 5 bis 25, insbesondere 7 bis 15 µm resultiert.

Als elektrisch leitende Träger sind prinzipiell alle leitfähigen Trägermaterialien verwendbar, soweit sie für das Einsatzgebiet geeignet sind. Bevorzugt sind je nach Einsatzgebiet der Aufzeichnungsmaterialien Aluminium-, Zink-, Magnesium-, Kupfer- oder Mehrmetallplatten, z.B. rohe oder vorbehandelte, z.B. aufgerauhte und/oder anodisierte Aluminiumbleche, Aluminiumfolien, Polymerfilme mit metallisierter Oberfläche wie aluminiumbedampfte Polyethylenterephthalatfilme oder auch elektrisch leitende Spezialpapiere. Träger für Druckformen haben vorteilhaft eine Dicke von 0,08 bis ca. 0,3 mm.

Die Art der geeigneten organischen Bindemittel für die Schichten richtet sich nach dem beabsichtigten Verwendungszweck der Aufzeichnungsmaterialien. Für den Kopiersektor eignen sich z.B. Celluloseether, Polyesterharze, Polyvinylchloride, Polycarbonate, Copolymere, wie Styrol-Maleinsäureanhydrid-Copolymere oder Vinylchlorid-Maleinsäure-anhydrid-Copolymere oder Mischungen solcher Bindemittel. Bei ihrer Auswahl spielen ihre filmbildenden und elektrischen Eigenschaften, ihre Haftfestigkeit auf dem Trägermaterial und ihre Löslichkeitseigenschaften eine besondere Rolle. Insbesondere bei Aufzeichnungsmaterialien für die Herstellung elektrophotographischer Druckplatten und besonders bei denen für den Offsetdruck sind solche besonders geeignet, die in basischen wässerigen oder alkoholischen Lösungsmitteln löslich sind. Dies sind vor allem Substanzen mit alkalilöslich machenden Gruppen wie Anhydrid-, Carboxyl-, Sulfonsäure-, Phenol- oder Sulfonimid-Gruppierungen. Bevorzugt sind Bindemittel, insbesondere solche mit hohen Säurezahlen, die in basischen wässerigalkoholischen Lösungsmittelsystemen leicht löslich sind und ein mittleres Molekulargewicht (Gewichtsmittel) von 800 bis 50 000 und insbesondere 1500 bis 10 000 aufweisen. So hat sich gezeigt, dass Copolymerisate aus Styrol, Maleinsäureanhydrid und Acryl- oder Methacrylsäure, die einen Anteil von Maleinsäureanhydrid von 15 bis 50 Gew.% und einen Anteil von Acryl- oder Methacrylsäure von bis zu 35 und insbesondere 10 bis 30 Gew.% aufweisen, befriedigende elektrophotographische Schichten mit hinreichender Dunkelleitfähigkeit ergeben. Sie weisen eine hervorragende Löslichkeit in Auswaschmitteln aus 75 Gew.% Wasser, 23 Gew.% Isobutanol und 2 Gew.% Soda auf, sind aber in offsettypischem Wischwasser unlöslich.

Geeignete Ladungsträger erzeugende Verbindungen bzw. Sensibilisatoren sind z.B. für einschichtig aufgetragene Systeme, wie sie auch zur Herstellung elektrophotographischer Druckformen dienen, Farbstoffe aus der Triarylmethanreihe, Xanthenfarbstoffe und Cyaninfarbstoffe. Sehr gute Ergebnisse wurden mit den erfindungsgemäss verwendeten Verbindungen der Formel (I) mit Rhodamin B (C.I. 45170), Rhodamin 6 G (C.I. 45160), Malachitgrün (C.I. Basic Green 4; C.I. 42000), Methylviolett (C.I. 42535) oder Kristallviolett (C.I. 42555) erhalten. Bei mehrschichtig aufgetragenen Systemen liegt der Farbstoff oder das Pigment in einer separaten Ladungsträger erzeugenden Schicht vor. Hier sind Azofarbstoffe, Phthalocyanine, Isoindolinfarbstoffe und Perylentetracarbonsäurederivate besonders wirksam. Gute Ergebnisse werden mit Perylen-3,4:9,10-tetracarbonsäurediimidderivaten erzielt, wie sie in den DE-A-31 10 954 und 31 10 960 beschrieben sind.

Für die jeweilige Verwendung kann das erfindungsgemässe elektrophotographische Aufzeichnungsmaterial übliche Zusätze enthalten, z.B. Verlaufmittel und Weichmacher in der photoleitfähigen Schicht oder Haftvermittler zwischen Träger und Schicht.

Die erfindungsgemässen elektrophotographischen Aufzeichnungsmaterialien zeichnen sich durch eine Kombination sehr guter Eigenschaften, insbesondere einer hohen Photoleitfähigkeit bei gleichzeitig sehr niedriger Dunkelleitfähigkeit aus, so dass die Schichten für die Kopiertechnik sehr geeignet sind. Deutliche Vorteile weisen sie bei der Verwendung für die Herstellung von elektrophotographischen Druckformen auf und genügen hierbei hohen Ansprüchen im Hinblick auf das Auflösungsvermögen und die Druckauflage.

Heterocyclenderivate wie Oxdiazolderivate (vgl. DE-B-1 058 836), Oxazolderivate (vgl. DE-B-1 120 875) oder 2,5-Bis-(4'-dialkylaminophenyl)-triazole-1,3,4 (vgl. DE-B-10 60 260) waren zwar als Ladungsträger transportierende Verbindungen bekannt, jedoch konnte sicher die mit den erfindungsgemäss verwendeten Triazolen erzielte Kombination vorteilhafter Eigenschaften nicht vorhergesehen werden. In der DE-B-27 37 334 wird zwar Benztriazol zusammen mit einem Bindemittel, einer reduzierbaren Metallverbindung und einem reduzierenden Mittel für die reduzierbare Metallverbindung in einer den elektrischen Strom leitenden Masse für ein wärmeentwickelbares Bildaufzeichnungselement verwendet, doch dürfte diese Literaturstelle die vorliegende Erfindung mit ihren Ergebnissen nicht nahelegen.

Die folgenden Beispiele sollen die Erfindung zusätzlich erläutern. Die genannten Teile und Prozente beziehen sich auf das Gewicht.

Die in den Beispielen angegebenen xerographischen Messgrössen A bis D werden wie folgt ermittelt:

Die Schichten werden mit einer Gleichspannungscorona von −7,5 kV in 1 cm Abstand gleichmässig auf ein Oberflächenpotential von 600 Volt aufgeladen und dann mit dem weissen Licht einer Xenonlampe mit einer Beleuchtungsstärke von etwa 0,85 mW $cm^{-2}$ belichtet. Es werden gemessen:

Messgrösse A: Zeit in Millisekunden (ms), innerhalb der das vor Belichtung vorhandene Oberflächenpotential bei aktinischer Belichtung um die Hälfte (300 V) abgefallen ist.

Messgrösse B: Potentialabfall in der gleichen Zeit in Volt (V), der im Dunkeln infolge Dunkelleitfähigkeit der Schichten eintritt.

Messgrösse C: Nach einer Beladungszeit von 20 Sekunden erreichtes Oberflächenpotential in Volt (V).

Messgrösse D: Potentialabfall in %, bezogen auf Messgrösse C, im Dunkeln innerhalb von 20 Sekunden.

*Beispiele 1 bis 5*

Auf eine Polyethylenterephthalatfolie mit einer aufgedampften, leitfähigen Aluminiumschicht in einer Dicke von etwa 300 Å wird eine Schicht aus 60 Teilen eines chlorierten Perylen-3,4:9,10-tetracarbonsäurediimidbisbenzimidazols mit einem Chlorgehalt von etwa 38% und 50 Teilen eines Copolymerisats aus Vinylchlorid, Acrylsäure und einem Maleinsäurediester in einer Dicke von etwa 0,55 µm als Ladungsträger erzeugende Schicht aufgebracht.

Auf diese Ladungsträger erzeugende Schicht wird aus einer Lösung in Essigsäureethylester eine Ladungstransportschicht aus 45 Teilen eines handelsüblichen Polycarbonat-Bindemittels mit einem Schmelzbereich von 220 bis 230° C, 10 Teilen eines Polyesters mit einer Säurezahl von etwa 40 und einem Molekulargewicht von ca. 4500 und 40 Teilen jeweils eines der nachstehend mit (1) bis (5) angegebenen Triazole so aufgebracht, dass nach dem Ablüften des Lösungsmittels und 30minütigem Trocknen bei 80° C eine Trockenschichtdicke von 12 µm resultiert.

Verwendete Triazole:

(1) 2-(4'-Diethylaminophenyl)-4,5-dimethyltriazol-1,2,3,
(2) 2-(4'-Diethylaminophenyl)-4-phenyl-5-carbmethoxytriazol-1,2,3,
(3) 2-(4'-Diphenylaminophenyl)-4,5-dimethyltriazol-1,2,3,
(4) 2-(4'-Dibenzylaminophenyl)-4,5-dimethyltriazol-1,2,3,
(5) 2-(4'-Dibenzylaminophenyl)-4-methyl-5-carbmethoxytriazol-1,2,3.

*Tabelle 1*

Xerographische Messgrössen der Aufzeichnungsmaterialien der Beispiele 1 bis 5.

| Beispiel | Triazol | Messgrösse | | |
|---|---|---|---|---|
| | | A (ms) | B (V) | C (V) |
| 1 | (1) | 320 | 0,25 | 1500 |
| 2 | (2) | 270 | 0,2 | 1600 |
| 3 | (3) | 190 | 0,3 | 1450 |
| 4 | (4) | 290 | 0,1 | 1250 |
| 5 | (5) | 250 | 0,12 | 1200 |

Wie die Messergebnisse zeigen, weisen die erfindungsgemässen elektrophotographischen Aufzeichnungsmaterialien eine hohe Photoleitfähigkeit und eine niedrige Dunkelleitfähigkeit auf. So zeigt z.B. die Schicht von Beispiel 1 im Dunkeln innerhalb von etwa 0,3 Sekunden einen Potentialabfall von 600 auf 599,9 Volt auf, während dieselbe Schicht in der gleichen Zeit bei Belichtung mit einer Beleuchtungsstärke von 0,85 mW $cm^{-2}$ von 600 auf 300 Volt abfällt. Die maximale Beladbarkeit ist mit grösser 1000 Volt weit über den in Kopiergeräten erforderlichen Oberflächenpotentialen (ca. 700 Volt), so dass die Schichten für die Kopiertechnik sehr geeignet sind.

*Beispiel 6*

Es wird wie in den Beispielen 1 bis 5 verfahren, jedoch wird die Ladungstransportschicht aus 55 Teilen eines Copolymerisats aus 80% Styrol und 20% Maleinsäureanhydrid und aus 45 Teilen 2-(4'-Dibenzylaminophenyl)-4-methyl-5-carbmethoxytriazol-1,2,3 hergestellt. Das Aufzeichnungsmaterial zeigt bei den xerographischen Messungen als Messgrösse D einen Potentialabfall um 12%, als Messgrösse A einen Potentialabfall um die Hälfte in 0,25 Sekunden. Wird diese Schicht als Kopierfolie in einem handelsüblichen Kopiergerät mit Trockentoner verwendet, so können damit Kopien von guter Qualität und in hoher Anzahl hergestellt werden.

*Beispiel 7*

Eine Lösung aus 45 Teilen 2-(4'-Dibenzylaminophenyl)-4-methyl-5-carbmethoxytriazol-1,2,3, 10 Teilen eines chlorierten Polyvinylchlorids, 50 Teilen eines Copolymerisats aus 55% Styrol, 40% Maleinsäureanhydrid und 5% Methacrylsäure und 0,3 Teilen Malachitgrün (C.I. 42000) wird auf eine durch Drahtbürstung mechanisch aufgerauhte 100 µm starke Aluminiumfolie mit einer Bürsttiefe von etwa 3 mm aufgetragen. Nach dem Verdunsten des Lösungsmittels hinterbleibt eine Photoleiterschicht mit einer Dicke von 4 bis 5 µm. Die Schicht wird in der in der Elektrophotographie üblichen Weise mit einer Gleichspannungscorona auf ein Oberflächenpotential von −400 V aufgeladen und bildmässig mit einer Xenonhochdrucklampe von 2 kW Leistung für 50 Sekunden belichtet. Das entstandene elektrostatische Bild der Vorlage wird durch Einstäuben mit einem durch Russ angefärbten Harzpulver sichtbar gemacht und durch Erwärmen auf 150° C zu einer wischfesten Elektrokopie fixiert. Man erhält so eine der Vorlage entsprechende Elektrokopie, welche an den Bildstellen gegen alkalische Lösungen beständig ist.

Zur Umwandlung der so hergestellten Elektrokopie in eine Flachdruckform wird mit einer Lösung behandelt, die aus 75% Wasser, 20% Isopropanol, 4,5% Natriumsilikat und 0,5% Soda besteht. Nach kurzem Spülen mit Wasser und Einfärben mit fetter Farbe können von der Druckform Abdrücke hergestellt werden.

## Patentansprüche

1. Elektrophotographische Aufzeichnungsmaterialien mit elektrisch leitenden Trägern, Ladungsträger erzeugenden Verbindungen und Ladungsträger transportierenden Verbindungen, dadurch gekennzeichnet, dass sie als Ladungsträger transportierende Verbindungen solche der Formel (I) enthalten

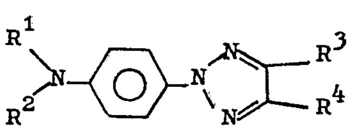 (I)

worin bedeuten:
$R^1$ und $R^2$ = Wasserstoff, Alkyl, Allyl, Benzyl oder ggf. subst. Phenyl, gleich oder verschieden, oder $R^1$ und $R^2$ gemeinsam

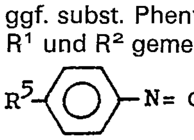 oder 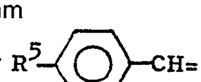
mit $R^5 = NR^1R^2$

$R^3$ = Alkyl oder Phenyl,
$R^4$ = Alkyl, Vinyl, Allyl, Carbalkoxy oder (Meth)-Acryloyl.

2. Elektrophotographische Aufzeichnungsmaterialien gemäss Anspruch 1, dadurch gekennzeichnet, dass in Formel (I) bedeuten
$R^1$ = Alkyl, Benzyl oder Phenyl
$R^2$ = Alkyl, Benzyl oder Phenyl
$R^3$ = Alkyl oder Phenyl
$R^4$ = Alkyl oder Carbalkoxy.

3. Elektrophotographische Aufzeichnungsmaterialien gemäss Anspruch 1, dadurch gekennzeichnet, dass sie einen elektrisch leitenden Träger, eine Schicht mit Ladungsträger erzeugenden Verbindungen und eine weitere Schicht mit Ladungsträger transportierenden Verbindungen gemäss der in Anspruch 1 gegebenen Formel (I) umfassen.

4. Elektrophotographische Aufzeichnungsmaterialien gemäss den Ansprüchen 1 oder 2, dadurch gekennzeichnet, dass sie aus einem elektrisch leitenden Träger und einer Schicht mit Sensibilisatoren oder Ladungsträger erzeugenden Verbindungen und Ladungsträger transportierenden Verbindungen gemäss der in Anspruch 1 gegebenen Formel (I) bestehen.

5. Verwendung der elektrophotographischen Aufzeichnungsmaterialien gemäss den Ansprüchen 1 bis 4 für reprographische Zwecke.

6. Verwendung der elektrophotographischen Aufzeichnungsmaterialien gemäss den Ansprüchen 1 bis 3 für die Herstellung von elektrophotographischen Druckplatten, insbesondere Offsetdruckplatten.

## Revendications

1. Matériaux d'enregistrement électrophotographiques à supports conducteurs d'électricité, composés générant des porteurs de charge et composés transportant des porteurs de charge, caractérisés par le fait qu'ils contiennent, comme composés transportant les porteurs de charge, ceux de formule (I)

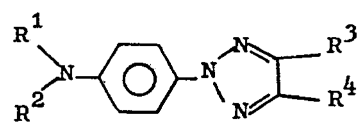 (I)

dans laquelle
$R^1$ et $R^2$ représentent hydrogène, alkyle, allyle, benzyle ou phényle éventuellement substitués, identiques ou différents, ou
$R^1$ et $R^2$ représentent ensemble

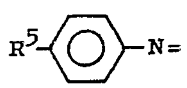 ou 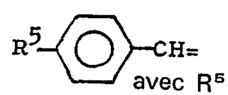
avec $R^5 = NR^1R^2$

$R^3$ représente alkyle ou phényle
$R^4$ représente alkyle, vinyle, allyle, carbalcoxy ou (meth)acryloyle.

2. Matériaux d'enregistrement électrophotographiques selon la revendication 1, caractérisés par le fait que, dans la formule (I)
$R^1$ = alkyle, benzyle ou phényle
$R^2$ = alkyle, benzyle ou phényle
$R^3$ = alkyle ou phényle
$R^4$ = alkyle ou carbalcoxy.

3. Matériaux d'enregistrement électrophotographiques selon la revendication 1, caractérisés par le fait qu'ils comprennent un support conducteur de l'électricité, une couche à composés générant des porteurs de charge et une autre couche à composés transportant des porteurs de charge selon la formule (I) donnée dans la revendication 1.

4. Matériaux d'enregistrement électrophotographiques selon les revendications 1 ou 2, caractérisés par le fait qu'ils sont constitués par un support conducteur de l'électricité et une couche à sensibilisateur ou composés générant des porteurs de charge et composés transportant des porteurs de charge selon la formule (I) donnée dans la revendication1.

5. Utilisation des matériaux d'enregistrement électrophotographiques selon les revendications 1 à 4, pour la reprographie.

6. Utilisation des matériaux d'enregistrement électrophotographiques selon les revendications 1 à 3, pour la fabrication de plaques d'impression électrophotographiques, en particulier de plaques offset.

## Claims

1. An electrophotographic recording material comprising an electrically conductive base, charge carrier-producing compounds and charge carrier-transporting compounds, which contains, as charge carrier-transporting compounds, those of the formula (I)

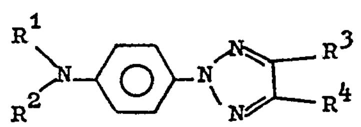 (I)

where
$R^1$ and $R^2$ are identical or different and are each hydrogen, alkyl, allyl, benzyl or unsubstituted or substituted phenyl, or $R^1$ and $R^2$ together are

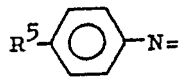 or 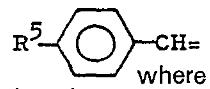 where $R^5$ is $NR^1R^2$
$R^3$ is alkyl or phenyl, and
$R^4$ is alkyl, vinyl, allyl, carbalkoxy, acryloyl or methacryloyl.

2. An electrophotographic recording material as claimed in claim 1, wherein, in formula (I), $R^1$ and $R^2$ are each alkyl, benzyl, or phenyl, $R^3$ is alkyl or phenyl, and $R^4$ is alkyl or carbalkoxy.

3. An electrophotographic recording material as claimed in claim 1, comprising an electrically conductive base, a layer containing charge carrier-producing compounds and another layer containing charge carrier-transporting compounds of the formula (I) given in claim 1.

4. An electrophotographic recording material as claimed in claim 1 or 2, consisting of an electrically conductive base and a layer containing sensitizers or charge carrier-producing compounds and charge carrier-transporting compounds of the formula (I) given in claim 1.

5. The use of an electrophotographic recording material as claimed in claims 1 to 4 for reprographic purposes.

6. The use of an electrophotographic recording material as claimed in claims 1 to 3 for the production of electrophotographic printing plates, in particular offset printing plates.